## Europäisches Patentamt
## European Patent Office
## Office européen des brevets

(19)

(11) Veröffentlichungsnummer: **0 150 770**
**B1**

(12) **EUROPÄISCHE PATENTSCHRIFT**

(45) Veröffentlichungstag der Patentschrift:
**15.10.86**

(21) Anmeldenummer: **85100427.5**

(22) Anmeldetag: **17.01.85**

(51) Int. Cl.⁴: **C 07 C 85/24**, C 07 C 87/58

(54) **Verfahren zur Orthoalkylierung von gegebenenfalls alkylsubstituierten m-Phenylendiaminen.**

(30) Priorität: **28.01.84 DE 3402983**

(43) Veröffentlichungstag der Anmeldung:
**07.08.85 Patentblatt 85/32**

(45) Bekanntmachung des Hinweises auf die Patenterteilung:
**15.10.86 Patentblatt 86/42**

(84) Benannte Vertragsstaaten:
**BE CH DE FR GB LI**

(56) Entgegenhaltungen:
**EP-A-0 069 065**
**DE-B-1 051 271**
**DE-B-1 056 138**

(73) Patentinhaber: **BAYER AG, Konzernverwaltung RP Patentabteilung, D-5090 Leverkusen 1 Bayerwerk (DE)**

(72) Erfinder: **Becker, Hans-Joachim, Dr., Elisabeth-Langgässer-Strasse 33, D-5090 Leverkusen (DE)**

EP 0 150 770 B1

## Beschreibung

Die Erfindung betrifft ein Verfahren zur Orthoalkylierung von gegebenenfalls alkylsubstituierten m-Phenylendiaminen.

Verfahren zur Herstellung von kernalkylierten aromatischen Aminen durch Umsetzung von aromatischen Aminen mit Olefinen in Gegenwart von Aluminium oder seinen Legierungen, wie Aluminiumamalgam oder Al/Ni-Legierung bzw. in Gegenwart der sich daraus bildenden Aluminiumanilide, bei erhöhten Temperaturen (200 - 350° C) und erhöhten Drücken (100 - 200 atm) sind seit langem bekannt (z.B. DE-PS 951 501).

Weiterhin ist bekannt (DE-AS 1 048 277), die Ausbeuten an kernalkylierten aromatischen Aminen unter gleichzeitiger Verkürzung der Reaktionszeit dadurch zu erhöhen, daß man bei der Alkylierung nach dem genannten Verfahren noch Friedel-Crafts-Katalysatoren, wie Aluminiumchlorid, oder Bleicherden zusetzt.

Ferner ist auch ein Zusatz von Jod oder Jodverbindungen zum Aluminium oder seinen Verbindungen bei der Kernalkylierung von aromatischen Aminen gemäß DE-PS 951 501 bekannt (siehe DE-AS 1 044 097).

Die Alkylierung von m-Phenylendiamin sowie von 2,4- und 2,6-Toluylendiamin mit Ethen wird speziell in der DE-AS 1 048 277 in den Beispielen 26 bis 31 sowie in Beispiel 34 beschrieben. So werden in den Beispielen 26 bis 28 die genannten Toluylendiamine in Gegenwart von Aluminiumchlorid und einer Lösung von Aluminiumanilid in Anilin mit Ethen bei 280 bis 300° C und einem Druck von 200 atm umgesetzt. Gemäß den Beispielen 29 und 30 wird in Gegenwart von Aluminium, Aluminiumchlorid, Quecksilberchlorid und Anilin gearbeitet und nach Beispiel 31 wird die Alkylierung in Gegenwart von Aluminium, Aluminiumchlorid und Quecksilberchlorid durchgeführt. Gemäß dem Beispiel 34 wird m-Phenylendiamin in Gegenwart einer Aluminiumanilid-Lösung und von wasserfreiem Aluminiumchlorid mit Ethen umgesetzt.

Die Orthoalkylierung der genannten m-Phenylendiamine wird außerdem auch in einer zusammenfassenden Arbeit (R. Stroh u. Mitarbeiter, Angew. Chem. 69, 124 - 131 (1957)) beschrieben. Als zweckmäßige Methode wird auch hier nur die Alkylierung unter Zusatz von Al-Anilid in Anilinlösung empfohlen.

Nachteilig bei den genannten Verfahren ist der Zusatz eines Hilfsamins in Form von Aluminiumanilid/Anilin-Lösung oder von Anilin, wodurch sich die Raum-Zeit-Ausbeute an gewünschtem Produkt erheblich verringert, die gleichzeitige Kernalkylierung des Hilfsamins, bei der in beträchtlichem Maße Nebenprodukte, wie 2-Ethylanilin, gebildet werden sowie die notwendige Abtrennung des restlichen Anilins und der Nebenprodukte, was einen erheblichen destillativen Aufwand erfordert (vgl. Beispiele 26 bis 30 und 34 der DE-AS 1 048 277). Weiterhin ist aus ökologischen Gründen der Zusatz von Quecksilberchlorid von Nachteil.

Es wurde nun ein Verfahren zur Orthoalkylierung von gegebenenfalls alkylsubstituierten m-Phenylendiaminen gefunden, das dadurch gekennzeichnet ist, daß man die entsprechenden m-Phenylendiamine mit einer Al/Zn-Legierung und Aluminiumchlorid erhitzt und nach Beendigung der Wasserstoffentwicklung mit niederen Alkenen bei erhöhten Drücken und Temperaturen umsetzt.

Als gegebenenfalls alkylsubstituierte m-Phenylendiamine können in das erfindungsgemäße Verfahren neben m-Phenylendiamin solche m-Phenylendiamine, die als Substituenten eine oder mehrere Alkyl-, Cycloalkyl- oder Aralkyl-Gruppen mit 1 bis 8, bevorzugt mit 1 bis 3 C-Atomen enthalten, wie Toluylendiamin-2,4, Toluylendiamin-2,6, 1-Ethylphenylendiamin-2,4, 1-Ethylphenylendiamin 2,6, 1-Propylphenylendiamin-2,4, 1,3-Dimethylphenylendiamin-4,6, 1-Benzylphenylendiamin-2,4, bevorzugt Toluylendiamin-2,4 und -2,6, eingesetzt werden. Die Phenylendiamine können sowohl einzeln als auch im Gemisch untereinander eingesetzt werden.

Als Al/Zn-Legierungen kommen solche mit einem Zinkgehalt von etwa 2 bis 30 Gew.-%, bevorzugt 5 bis 20 Gew.-%, in Frage. Der Aluminiumgehalt beträgt entsprechend etwa 70 bis 98 Gew.-%, bevorzugt 80 bis 95 Gew.-%. Die Legierungen können in Form von Drehspänen, von Granulaten, von Grieß oder von Pulver eingesetzt werden.

Die Menge der einzusetzenden Al/Zn-Legierung kann variiert werden und hängt u.a. vom Zn-Anteil in der Al/Zn-Legierung ab. Im allgemeinen werden etwa 0,5 bis 4 Gew.-%, bevorzugt 1 bis 2 Gew.-%, Al/Zn-Legierung, bezogen auf das zu alkylierende m-Phenylendiamin, eingesetzt.

Die Menge des eingesetzten Aluminiumchlorids beträgt üblicherweise etwa 1 bis 7 Gew.-%, bevorzugt 2,5 bis 4 Gew.-%, bezogen auf das zu alkylierende m-Phenylendiamin.

Nach dem erfindungsgemäßen Verfahren wird zunächst das zu orthoalkylierende gegebenenfalls alkylsubstituierte m-Phenylendiamin mit einer Al/Zn-Legierung und dem Aluminiumchlorid (wasserfrei) so lange bei Temperaturen von etwa 150 bis 250° C, bevorzugt 170 bis 220° C, erhitzt bis keine Wasserstoffentwicklung mehr zu beobachten ist. Dann wird das Gemisch im Autoklaven bei Temperaturen von etwa 250 bis 330° C, bevorzugt 280 bis 310° C, mit dem niederen Alken umgesetzt. Die Drücke betragen dabei etwa 50 bis 250 bar, bevorzugt 80 bis 120 bar. Nach Beendigung der Alkenaufnahme wird noch etwas nachreagieren lassen und nach dem Entspannen des überschüssigen Alkens das Reaktionsgemisch aufgearbeitet.

Als niedere Alkene können in das erfindungsgemäße Verfahren Ethen und Propen, bevorzugt Ethen, eingesetzt werden.

Die Aufarbeitung des Reaktionsgemisches erfolgt in bekannter Weise durch Versetzen des Reaktionsgemisches mit Wasser oder mit wäßriger Natronlauge, wodurch der Katalysator zersetzt wird. Die organische Phase kann anschließend einer Vakuumdestillation unterworfen werden.

Nach dem erfindungsgemäßen Verfahren können die m-Phenylendiamine in etwa 96 bis 98 %iger Ausbeute orthoalkyliert werden. Wie bereits erwähnt, wird gegenüber einem Verfahren mit Zusatz von Aluminiumanilid und Anilin oder ähnlichen tiefsiedenden aromatischen Monoaminen bei dem erfindungsgemäßen Verfahren die Raum-Zeit-Ausbeute beträchtlich erhöht und der Destillationsaufwand erheblich vermindert. Die Wirtschaftlichkeit der Orthoalkylierung wird dadurch gegenüber den dem Stand der Technik entsprechenden Verfahren wesentlich gesteigert.

Da gemäß dem Beispiel 10 der DE-PS 951 501 bei der Umsetzung von Anilin und Ethen bei Verwendung einer Al/Ni-Legierung trotz scharfer Reaktionsbedingungen nur ein Umsatz von ca. 25% erreicht wurde, mußte auch bei der Orthoalkylierung von gegebenenfalls alkylsubstiuierten m-Phenylendiaminen mit Ni/Al-Legierungen mit negativen Ergebnissen gerechnet werden. In der Tat wird auch bei der Umsetzung von Toluylendiamin und Ethen in Gegenwart einer Al/Ni-Legierung, ebenso wie beim Einsatz von reinem Aluminium (vgl. Beispiel 1), keine nennenswerte Wasserstoffentwicklung und keinerlei Alkylierungsreaktion beobachtet. Ebenfalls schlechte Ergebnisse wurden bei der Orthoalkylierung von Toluylendiaminen mit Aluminiumlegierungen, die z.B. Magnesium, Zinn, Blei, Cadmium oder Eisen enthalten, beobachtet. Es ist deshalb besonders überraschend, daß gerade eine Al/Zn-Legierung gute Ergebnisse bei der Orthoalkylierung von gegebenenfalls alkylsubstituierten m-Phenylendiaminen liefert.

Insgesamt kann festgestellt werden, daß nach dem erfindungsgemäßen Verfahren die Orthoalkylierung von gegebenenfalls alkylsubstituierten m-Phenylendiaminen mit Al/Zn-Legierung und Aluminiumchlorid in ebenso einfacher Weise durchgeführt werden kann, wie die der Monoamine mit Aluminium und Aluminiumchlorid.

Die orthoalkylierten m-Phenylendiamine eignen sich besonders als Vernetzerkomponente für Polyurethankunststoffe (siehe DE-AS 2 622 951).

Die nachfolgenden Beispiele sollen das erfindungsgemäße Verfahren verdeutlichen, ohne es jedoch auf die Beispiele einzuschränken.

**Beispiel 1**

250 g Toluylendiamin 65/35 (Gemisch aus 65 Gew.-% Toluylendiamin-2,4 und 35 Gew.-%

Toluylendiamin-2,6) werden mit 4,7 g einer Al/Zn-Legierung, bestehend aus 70 Gew.-% Al und 30 Gew.-% Zn, und 8,3 g Aluminiumchlorid (wasserfrei) unter Rühren auf 200°C (maximal 220°C) erhitzt. Die Wasserstoffentwicklung beginnt bei 150°C, sie ist nach etwa 45 Minuten beendet.

In einem Rührautoklaven wird der Reaktionsansatz anschließend bei Temperaturen von 290 - 300°C und bei einem Druck von 200 bar mit Ethen umgesetzt. Die Ethenaufnahme ist nach 25 - 30 Minuten beendet.

Nach Aufarbeitung mit wäßriger Natronlauge erhält man ein Alkylierungsprodukt, das nach gaschromatographischer Analyse 98 % an dialkylierten Toluylendiaminen (1-Methyl-3,5-diethylphenylendiamin-2,4 und 1-Methyl-3,5-diethylphenylendiamin-2,6) enthält.

Bei Einsatz von reinem Aluminium oder einer Al/Ni-Legierung anstelle der Al/Zn-Legierung findet keine nennenswerte Wasserstoffentwicklung und Alkylierung statt.

**Beispiel 2**

250 g Toluylendiamin 65/35 werden wie in Beispiel 1 beschrieben, mit 4,7 g einer Al/Zn-Legierung (70 Gew.-% Al, 30 Gew.-% Zn) und 4,15 g Aluminiumchlorid zur Reaktion gebracht.

Die Ethylierung analog Beispiel 1 ist nach 45 Minuten beendet und ergibt ein Alkylierungsprodukt mit einem Gehalt von 98,4 % an den in Beispiel 1 erwähnten Diethyl-Verbindungen.

**Beispiel 3**

250 g Toluylendiamin 65/35 werden gemäß Beispiel 1 mit 4,1 g einer Al/Zn-Legierung, bestehend aus 80 Gew.-% Aluminium und 20 Gew.-% Zn, und 8,3 g Aluminiumchlorid (wasserfrei) umgesetzt. Die Wasserstoffentwicklung ist nach 22 Minuten beendet.

Das Alkylierungsprodukt, das analog Beispiel 1 hergestellt wurde, enthält 97,3 % Diethyltoluylendiamine.

**Beispiel 4**

Bei einem Einsatz von 250 g Toluylendiamin 65/35, 8,3 g Aluminiumchlorid (wasserfrei) und 3,65 g einer Al/Zn-Legierung (90 Gew.-% Al/lO Gew.-% Zn) bzw. 3,45 g Al/Zn-Legierung (95 Gew.-% A1/5 Gew.-% Zn) werden jeweils Alkylierungsprodukte mit einem Gehalt von 96,9 % an den beiden Diethyltoluylendiaminen erhalten.

## Beispiel 5

Toluylendiamin-2,4 wurde analog Beispiel 1 mit Ethen umgesetzt. Das Alkylierungsprodukt enthält 96,9 % 1-Methyl-3,5-diethylphenylendiamin-2,4. Die Reaktionszeit beträgt 45 Minuten.

## Beispiel 6

Bei Einsatz von Toluylendiamin-2,6 analog Beispiel 5 wird ein Alkylierungsprodukt mit 97,3 % an 1-Methyl-3,5-diethylphenylendiamin-2,6 erhalten. Die Reaktionszeit beträgt 25 Minuten.

## Beispiel 7

Analog Beispiel 1 wird ein Gemisch bestehend aus 80 Gew.-% Toluylendiamin-2,4 und 20 Gew.-% Toluylen-diamin-2,6 mit Ethen umgesetzt. Man erhält ein Alkylierungsprodukt mit einem Gehalt von 95,5 % an den entsprechenden Diethyltoluylendiaminen.

## Patentansprüche

1. Verfahren zur Orthoalkylierung von gegebenenfalls alkylsubstituierten m-Phenylendiaminen, dadurch gekennzeichnet, daß man die entsprechenden m-Phenylendiamine mit einer Al/Zn-Legierung und Aluminiumchlorid erhitzt und nach Beendigung der Wasserstoffentwicklung mit niederen Alkenen bei erhöhten Drücken und Temperaturen umsetzt.

2. Verfahren nach Anspruch 1, dadurch gekennzeichnet, daß man eine Al/Zn-Legierung einsetzt, die einen Zinkgehalt von 2 bis 30 Gew.-% und einen Aluminiumgehalt von 70 bis 98 Gew.-% besitzt.

3. Verfahren nach Ansprüchen 1 und 2, dadurch gekennzeichnet, daß man eine Al/Zn-Legierung einsetzt, die einen Zinkgehalt von 5 bis 20 Gew.-% und einen Aluminiumgehalt von 80 bis 95 Gew.-% besitzt.

4. Verfahren nach Ansprüchen 1 bis 3, dadurch gekennzeichnet, daß man 0,5 bis 4,0 Gew.-% Al/Zn-Legierung, bezogen auf das zu alkylierende m-Phenylendiamin, einsetzt.

5. Verfahren nach Ansprüchen 1 bis 4, dadurch gekennzeichnet, daß man 1,0 bis 7,0 Gew.-% Aluminiumchlorid, bezogen auf das zu alkylierende m-Phenylendiamin einsetzt.

6. Verfahren nach Ansprüchen 1 bis 5, dadurch gekennzeichnet, daß man die Umsetzung mit dem Alken bei Drücken von 50 bis 250 bar durchführt.

7. Verfahren nach Ansprüchen 1 bis 6, dadurch gekennzeichnet, daß man die Umsetzung mit dem Alken bei Temperaturen von 250 bis 330°C durchführt.

## Claims

1. Process for the ortho-alkylation of optionally alkyl-substituted m-phenylenediamines, characterised in that the appropriate m-phenylenediamines are heated with an Al/Zn alloy and aluminium chloride and are reacted with lower alkenes at elevated pressures and temperatures when the evolution of hydrogen is complete.

2. Process according to Claim 1, characterised in that an Al/Zn alloy which has a zinc content of 2 to 30% by weight and an aluminium content of 70 to 98% by weight is employed.

3. Process according to Claims 1 and 2, characterised in that an Al/Zn alloy which has a zinc content of 5 to 20% by weight and an aluminium content of 80 to 95% by weight is employed.

4. Process according to Claims 1 to 3, characterised in that 0.5 to 4.0% by weight of Al/Zn alloy, relative to the m-phenylenediamine to be alkylated, are employed.

5. Process according to Claims 1 to 4, characterised in that 1.0 to 7.0% by weight of aluminium chloride, relative to the m-phenylenediamine to be alkylated, are employed.

6. Process according to Claims 1 to 5, characterised in that the reaction with the alkene is carried out under pressures of 50 to 250 bar.

7. Process according to Claims 1 to 6, characterised in that the reaction with the alkene is carried out at temperatures of 250 to 330°C.

## Revendications

1. Procédé d'ortho-alkylation de m-phénylène-diamines éventuellement substituées par un groupe alkyle, caractérisé en ce qu'on chauffe les m-phénylène-diamines correspondantes avec un alliage d'Al/Zn et du chlorure d'aluminium tandis que, au terme du dégagement d'hydrogène, on les fait réagir avec des alcènes inférieurs à des températures élevées et sous de fortes pressions.

2. Procédé selon la revendication 1, caractérisé en ce qu'on utilise un alliage d'Al/Zn ayant une teneur en zinc de 2 à 30% en poids et une teneur en aluminium de 70 à 88% en poids.

3. Procédé selon les revendications 1 et 2, caractérisé en ce qu'on utilise un alliage d'Al/Zn ayant une teneur en zinc de 5 à 20% en poids et une teneur en aluminium de 80 à 85% en poids.

4. Procédé selon les revendications 1 à 3, caractérisé en ce qu'on utilise 0,5 à 4% en poids d'un alliage d'Al/Zn, calculé sur la m-phénylène-diamine à alkyler.

5. Procédé selon les revendications 1 à 4, caractérisé en ce qu'on utilise 1 à 7% en poids de chlorure d'aluminium, calculé sur la mphénylène- diamine à alkyler.

6. Procédé selon les revendications 1 à 5, caractérisé en ce qu'on effectue la réaction avec l'alcène sous des pressions de 50 à 250 bars.

7. Procédé selon les revendications 1 à 6, caractérisé en ce qu'on effectue la réaction avec l'alcène à des températures de 250 à 330°C.